# EUROPEAN PATENT SPECIFICATION

(11) **EP 3 715 110 B1**
(45) Date of publication and mention of the grant of the patent: **17.04.2024**
(21) Application number: 19382224.4
(22) Date of filing: 28.03.2019
(51) Int. Cl.: B32B 7/12, B32B 23/04, B32B 23/20, C12P 7/625, C12P 19/04, C12P 39/00, B32B 27/36, B32B 23/08, A43B 1/00

(54) **LEATHER ALTERNATIVE MATERIAL**
LEDERALTERNATIVMATERIAL
MATÉRIAU ALTERNATIF AU CUIR

(43) Date of publication of application: 30.09.2020
(60) Divisional application: 24162144.0
(73) Proprietor: Next-Gen Leather, S.L., 28010 Madrid (ES)
(72) Inventor: GARCÍA MARTÍNEZ, Concepción, 28010 Madrid (ES)
(74) Representative: ABG Intellectual Property Law, S.L.

(56) References cited:
- EP-A1- 2 540 182
- EP-A1- 3 266 608
- EP-A2- 0 514 137
- EP-B1- 0 514 137
- WO-A1-99/01278
- US-A1- 2013 004 784
- US-A1- 2013 004 784
- US-A1- 2018 148 890
- GARCÍA CONCHA ET AL: "Bacterial cellulose as a potential bioleather substitute for the footwear industry", MICROBIAL BIOTECHNOLOGY, [Online] vol. 12, no. 4, 22 August 2018 (2018-08-22), pages 582-585, XP093013552, GB ISSN: 1751-7915, DOI: 10.1111/1751-7915.13306 Published online 2018 Aug 22 Retrieved from the Internet: URL:https://onlinelibrary.wiley.com/doi/fu ll-xml/10.1111/1751-7915.13306> [retrieved on 2023-01-13]

## Description

### TECHNICAL FIELD OF THE INVENTION

The present invention may be encompassed within the field of leather and related industries. More particularly, it relates to a sustainable material that can be used as a leather substitute, to the preparation method for preparing said material, to a footwear or any other article comprising same and to the use thereof as a structural component in industries associated with leather such as the textile, clothing, apparel, footwear, furniture and transport sectors.

### BACKGROUND OF THE INVENTION

Leather, in its most traditional definition, is the material that results from tanning animal's hides or skins. The leather manufacturing process may be divided into three fundamental subprocesses, namely the beamhouse (preparation of hides or skins), tanning (in the tanyard) and finishing (in post tanning area). More specifically, the process involves a series of mechanical and chemical operations which may be systematized as follows: soaking, unhairing and liming, fleshing and splitting, deliming and bating, pickling, tanning, neutralization, retanning, dying, fatliquoring, drying milling, and lastly finishing. Environmental issues associated with these operations include wastewater, air emissions, solid waste and hazardous materials. Further, raising and slaughtering the millions of animals whose skins feed the industry is inefficient, cruel, and comes with a huge environmental cost. Nowadays, both sustainability and animal welfare have become increasingly important issues for the society.

With these concerns in mind, synthetic substitutes for leather have gained interest over the last years. Firstly produced in the early 1900s, synthetic leather only became popular in the 1960s as the manufacturing process was improved, and since then it has gradually replaced genuine leather in many fields. Synthetic leather (also commonly known as faux, artificial or imitation leather) is not made from animal skin or hide like genuine leather, but from natural and/or synthetic fibres, coated with a plastic polymer or similar. Though there are a number of materials used, most synthetic leathers consist of a knitted polyester base with a polyurethane (PU) and polyvinyl chloride (PVC) coating. Processing of these plastic-based products is still far from being environmentally friendly.

Therefore, current trends move towards the use of non-petroleum-based materials. Synthetic leather has been already produced from vegetable sources such as apples (The Apple Girl), pineapples (Ananas Anam), grapes (VEGEA), mushrooms (Grado Zero Espace), soy (XXLab), paper (Paper No. 9), corn (Coronet), cork (Pelcor) and tea (Iowa University).

Cellulose is the most abundant material in the world. It is a naturally occurring polymer in plants, but which can also be obtained from fungi, algae, and bacteria through biosynthetic processes. Most of the cellulose used in the industry is of vegetable origin; however, in the last twenty years, cellulose of bacterial origin has taken importance due to its excellent physical properties compared to that obtained naturally by plants, which make it attractive for specific applications. Bacterial cellulose (BC) has a wide variety of applications in the paper, textile, food, explosive and fermentable sugar industries, among others, in addition to specific applications in medicine and biotechnology, as well as in the manufacture of membranes used as separation agents.

BC is an extracellular biopolymer produced by different bacteria through a fermentation process, bacteria, especially those belonging to the genus *Komagataeibacter* (formerly known as *Acetobacter* and later renamed as *Gluconoacetobacter*)*.* Under conditions of static culture, BC is produced as a gelatinous film whose 3D arrangement is pure cellulose nanofibrils with a structure formed by interconnected pores. One of the most important characteristics of BC is its purity, which distinguishes it from the vegetable cellulose usually associated with hemicellulose and lignin. Another feature is its high degree of crystallinity (greater than 60%) which, together with its 3D nanofibrillary architecture, makes it highly resistant to traction. It also has a high degree of polymerization, high water retention capacity and once purified, BC is non-toxic, non-allergenic, biocompatible and biodegradable.

Various studies show that this material has characteristics to be used in the leather industry as an alternative to natural and synthetic leather. The results obtained allow to validate its use as a material for footwear and related accessories and its possible industrial production, demonstrating that it is possible to produce fabrics from bacteria.

However, despite these excellent properties, the hydrophilic nature and liquid-absorb capacity of BC represent an important drawback for the leather industry, which demands water resistant materials that stay dry under raining conditions or liquid spills and not materials that get wet easily, absorbing water or liquids and swelling.

US2018148890A1 discloses synthetic leather materials and methods of making and use thereof such as in the preparation of shoes. The methods of making the synthetic leather materials comprise: synthesizing a piece of cellulose from a microbe, thereby forming a piece of microbial cellulose; partially drying the piece of microbial cellulose; treating the partially dried piece of microbial cellulose with a conditioning agent (e.g. PEG), thereby forming a piece of conditioned microbial cellulose; drying the piece of conditioned microbial cellulose; and treating the dried piece of conditioned microbial cellulose with a hydrophobic agent, thereby forming the synthetic leather material.

There is thus still a need for new ecofriendly leather substitutes.

### BRIEF DESCRIPTION OF THE INVENTION

The present invention provides a sheet-like material based on bacterial cellulose (BC) that can be used as a leather substitute. The material proposed is respectful with the environment and may be advantageously obtained by means of an easy and cost-effective process applicable at industrial scale that avoids the use of animals and highly pollutant chemicals. In particular, the inventors have found that the attachment of a biobased and biodegradable plastic to a layer of BC results in a water resistant layered material suitable for the leather industry. Such a bioplastic gives waterproofing properties to the BC without interfering with its good mechanical properties. Additionally, the bioplastic layer may also provide the characteristic shine of a patent leather finish.

In an aspect, the present invention relates to a footwear which comprises a material comprising:
- a layer of bacterial cellulose (BC); and
- a layer of a biobased and biodegradable plastic selected from the group consisting of polyesters or copolymers thereof;
wherein both layers are attached each other.

In another aspect the present invention relates to a method for preparing the footwear of the invention as above defined, said method comprising the preparation of the material by a process which comprises the following steps:
- providing a layer of bacterial cellulose (BC);
- providing a layer of biobased and biodegradable plastic selected from the group consisting of polyesters or copolymers thereof; and
- attaching both layers.

Further aspects of the invention relate to the use of the material of the invention as leather substitute.

These aspects and preferred embodiments thereof are additionally also defined hereinafter in the detailed description and in the claims.

### BRIEF DESCRIPTION OF THE FIGURES

To better understand the invention, its objects and advantages, the following figures are attached to the specification in which the following is depicted:

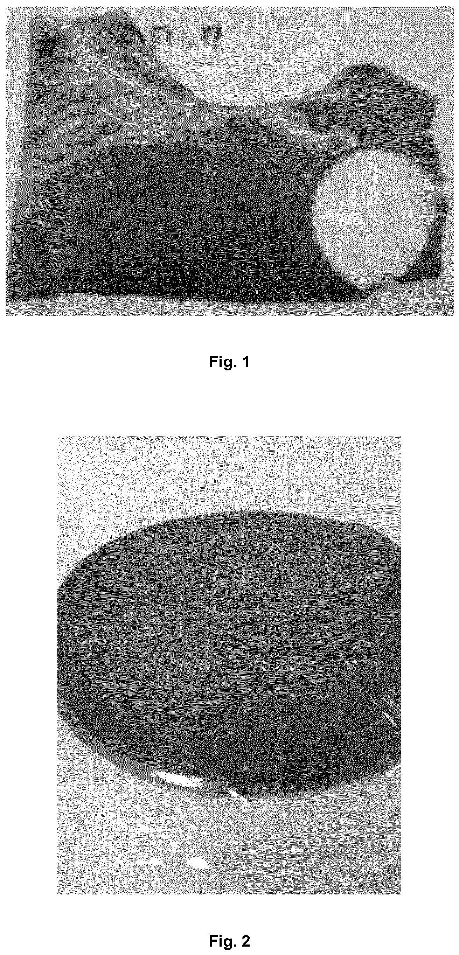

**Figure 1** is a picture of a sample of material according to the present invention subjected to the sessile drop wettability test. The bioplastic layer was a medium chain length polyhydroxyalkanoate (mcIPHA) with repeating units of C8, C10 and C12 monomers. As may be appreciated, the material was highly resistant to water.
**Figure 2** is a picture of a sample of material according to the present invention subjected to the sessile drop wettability test. The bioplastic layer was poly(lactic acid) (PLA). As may be appreciated, the material was highly resistant to water.

### DETAILED DESCRIPTION OF THE INVENTION

Unless defined otherwise, all technical and scientific terms and expressions used herein have the same meaning as commonly understood to one of ordinary skill in the art to which this disclosure belongs.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

By "room temperature" or its abbreviation "rt" is meant herein that the reactions or processes are performed without heating or cooling. Generally, by room temperature may be understood as a temperature between about 15 °C and about 30 °C, or more particularly between about 20 °C and about 25 °C.

Here, "sheet-like" refers to a shape whose average size in two spatial directions is much larger than the size of the third spatial direction which is orthogonal to the other spatial directions. Put another way, the term "sheet-like" refers to all forms of space whose thickness is considerably less than its length and width.

Biomaterial or bio-based material (e.g. bioplastic or bio-based plastic) refers to a material whose main constituent consists of a substance, or substances, originally derived from living organisms (e.g. plants, microbes), or components of them (e.g. enzymes), or biochemicals (i.e. chemicals found naturally in the living organisms such as sucrose, glucose, starch or natural polyesters). For example, cellulose can be obtained from plants as well as from fungi, algae, and bacteria through biosynthetic processes; starch can be processed to produce lactic acid and subsequently polylactic acid (PLA); polyhydroxyalkanoates (PHAs) are polyesters produced in nature by numerous microorganisms, including through bacterial fermentation of sugar or lipids.

Biodegradable material (e.g. biodegradable plastic) refers to a material which degrades under biological (mainly microbial) action. Some biodegradable materials are compostable (but not all), which means they degrade under aerobic conditions usually within a time frame of about 6-12 weeks. Composting of industrial products usually takes place in industrial composting plants, where controlled conditions (e.g. temperature, humidity, aeration) are given. Microbes, like bacteria or fungi and their enzymes, are able to "digest" the chain structure of compostable polymers as a source of nutrition. The resulting end products are water, carbon dioxide CO₂ and a little biomass. For instance, cellulose, PHA and PLA biopolymers are biobased, biodegradable and can be considered compostable.

For the purposes of the present invention, compostable materials preferably meet specific requirements of compostability according to at least one accepted standardization system such as ASTM 6400, ASTM 6868, ISO 17088, ISO 18606, EN 13432, EN 14995 or at least one accepted certification system such as certificates for compostable plastics issued by DIN Certco, Vincotte, Biodegradable Products Institute (BPI, US), the Japan BioPlastics Association (JBPA, Japan) as well as other less widely used organizations. More particularly, such standardization and certification systems correspond to the latest version as in force at the priority date of the present patent application.

The material of the invention is a sheet-like material suitable as leather substitute. The leather-like sheet as herein proposed comprises at least the following layers:
- bacterial cellulose (BC);
- bioplastic selected from polyesters [e.g. polyhydroxyalkanoate (PHA), poly(lactic acid) (PLA)] or a copolymer thereof;
wherein both layers are bonded each other e.g. by means of an intermediate layer or through any chemical or mechanical modification of said layers.

Preferably, the substances that made up the leather-like material of the invention are biobased or biodegradable. More preferably, the substances that made up the leather-like material of the invention are biobased and biodegradable. Even more preferably, the substances that made up the leather-like material of the invention are biobased and compostable.

Cellulose produced by bacteria, has grown in popularity since its discovery in 1886 due to its special properties, such as high purity, an ultrafine and highly crystalline network structure, a superior mechanical strength, biodegradability, biocompatibility, large water-holding capacity (WHC) and good chemical stability. BC is over 10-times stronger than plant-based cellulose. It is currently used to create materials for tissue engineering, medicine, defense, electronics, acoustics, and fabrics.

BC can be produced in different forms based on different fermentation conditions. Many sugars and sugar alcohols can be utilized as carbon source, the most important fermentation source, for BC production. Several studies have been developed to produce BC on an industrial scale, with a continuous or semicontinuous process, low-cost raw materials and small production of by-products.

In the present invention, BC may be obtained according to any known procedure in the state of the art such as those described for instance in or in the references cited therein: Florea, M., Hagemann, H., Santosa, G., Abbott, J., Micklem, C.N., Spencer-Milnes, X., et al. (2016) Engineering control of bacterial cellulose production using a genetic toolkit and a new cellulose-producing strain. Proc Natl Acad Sci USA 113, E3431-E3440. Islam, M.U., Ullah, M.W., Khan, S., Shah, N., and Park, J.K. (2017) Strategies for cost-effective and enhanced production of bacterial cellulose. Int J Biol Macromol 102: 1166-1173. Kubiak, K., Kurzawa, M., Jezdrzejczak-Krzepkowska, M., Ludwicka, K., Krawczyk, M., Migdalski, A., et al. (2014) Complete genome sequence of Gluconacetobacter xylinus E25 strain-valuable and effective producer of bacterial nanocellulose. J Biotechnol 20: 18-19. Lee, K.Y., Buldum, G., Mantalaris, A., and Bismarck, A. (2014) More than meets the eye in bacterial cellulose: biosynthesis, bioprocessing, and applications in advanced fiber composites. Macromol Biosci 14: 10-32. Andréa Fernanda De Santana Costa, Maria Alice Vasconcelos Rocha, and Leonie Asfora Sarubbo. (2017) Review - Bacterial Cellulose: An Ecofriendly Biotextile. International Journal of Textile and Fashion Technology (IJTFT) 7: 11-26. Campano, C., Balea, A., Blanco, A. et al. (2016) Enhancement of the fermentation process and properties of bacterial cellulose: a review. Cellulose 23: 57-91. Costa, A., Almeida, F., Vinhas, G. M., & Sarubbo, L. A. (2017). Production of Bacterial Cellulose by Gluconacetobacter hansenii Using Corn Steep Liquor As Nutrient Sources. Frontiers in microbiology 8: 2027. Faezah Esa, Masrinda Tasirin, Norliza Abd Rahman (2014) Overview of Bacterial Cellulose Production and Application. Agriculture and Agricultural Science Procedia 2: 113-119. Moniri, M., Boroumand Moghaddam, A., Azizi, S., Abdul Rahim, R., Bin Ariff, A., Zuhainis Saad, W., Navaderi, M., Mohamad, R. (2017). Production and Status of Bacterial Cellulose in Biomedical Engineering. Nanomaterials, 7(9): 257. Picheth GF, Pirich CL, Sierakowski MR, Woehl MA, Sakakibara CN, de Souza CF, Martin AA, da Silva R, de Freitas RA. (2017) Bacterial cellulose in biomedical applications: A review. Int J Biol Macromol. 104: 97-106. Kaiyan Qiu & Anil N. Netravali (2014) A Review of Fabrication and Applications of Bacterial Cellulose Based Nanocomposites, Polymer Reviews, 54:4, 598-626. Reiniati I, Hrymak AN, Margaritis A. (2017) Recent developments in the production and applications of bacterial cellulose fibers and nanocrystals. Crit Rev Biotechnol. 37(4):510-524.

In a particular embodiment of the invention, the method for producing BC comprises (i) culturing in a culture medium one or more bacterial strains which are capable of accumulating BC and (ii) recovering the BC produced from the culture medium.

BC may be for instance produced by bacteria of the genera *Komagataeibacter* (formerly known as *Acetobacter* and later renamed as *Gluconoacetobacter*)*, Agrobacterium, Achromobacter, Aerobacter, Enterobacter, Sarcina, Rhizobium, Pseudomonas, Salmonella, Alcaligenes and Myxedema.* In a more particular embodiment, BC is obtained by bacteria of the genus *Komagataeibacter* such as *Komagataeibacter xylinus, Komagataeibacter hansenii, Komagataeibacter kombuchae, Komagataeibacter intermedius.* Even more particularly, the BC-producing microorganism is a bacterial strain of *Komagataeibacter xylinus.* Important strains useful of *K. xylinus* for the production of BC include, but are not limited to, K3, BPR 2001, KU1, FC01, ATCC 53524, and MTCC 2623.

In a preferred embodiment, BC is obtained from a symbiotic culture of bacteria and yeast (SCOBY) comprising at least one type of BC-producing bacteria and at least one yeast type. Suitable bacteria for the SCOBY include, but are not limited to, those mentioned in the previous paragraph. More preferably, the symbiotic culture comprises at least one bacterial strain of the genus *Komagataeibacter,* preferably of the species *Komagataeibacter xylinus.* Suitable yeasts for the SCOBY include, but are not limited to, *Zygosaccharomyces rouxii* and *Candida sp.* Even more preferably, the symbiotic culture comprises at least one bacterial strain of the genus *Komagataeibacter,* preferably of the species *Komagataeibacter xylinus,* and the yeasts *Zygosaccharomyces rouxii* and *Candida sp.*

Culture media suitable for the production of BC are well known in the art. Usually said culture media comprise carbon sources, such as, for example, glucose, sucrose, fructose, glycerin, mannitol or arabitol and suitable nitrogen sources such as, for example, yeast extract and peptone.

In a particular embodiment, the SCOBY may be cultured in a medium comprising tea infusion like black or green tea as a source of nitrogen and a sugar (e.g. sucrose) as carbon source. In a more particular embodiment, the process for producing BC may be as follows: a black or green tea infusion is prepared by placing tea leaves in boiling water (e.g. about 2 g to about 6 g of tea leaves per liter of water). After about 10-20 minutes, tea leaves are withdrawn and the carbon source is added (e.g. 70 - 90 g/l sucrose). When the temperature decreases (e.g. below about 30 °C), the mixture is thrown into a bucket, such as one of about 0.09 m² (i.e. 1 square foot) in area which is the standard piece used in footwear industry. Then, the SCOBY is added in suspension (e.g. 5% - 15% v/v) and the buckets are covered with a filter such as filter paper. Fermentation normally takes place in about 10 - 20 days at a temperature of about 25 °C - 35 °C. Then, the sheets of BC are taken and washed with water or soapy water. Preferably, several washes are carried out, after which the sheets of BC may be purified by known procedures: for instance, the sheets may be immersed in a 2% potassium hydroxide solution for about 60 - 120 minutes at about room temperature with oscillating agitation for the elimination of impurities and cell debris, followed by a neutralization treatment with acid (e.g. acetic acid) for about 15 to 45 minutes at about room temperature. Subsequently, the sheets of BC may be dried vertically for about 24 hours at about 35 °C.

Instead of small buckets (e.g. 30 × 30 cm which approximately corresponds to 1 square foot) it is possible to use larger containers to obtain large-format BC sheets which may be subsequently divided up into individual sheets of the desired size.

Typically, the average thickness of the BC layer ranges from about 0.2 mm to about 1.2 mm (e.g. about 0.3, 0.4, 0.5, 0.6, 0.7, 0.8, 0.9, 1.1 mm). In a particular embodiment, the BC layer is about 0.6 mm in average thickness.

The layer/sheet of bacterial cellulose may be optionally dyed. A wide variety of commercial dyes are available in the leather industry and applicable in the present invention including for instance azo dyes and natural dyes. Preference is given to ecofriendly and non-toxic dyes that can be used to provide a full variety of colors. In a preferred embodiment, the BC is colored with a natural dye such as for instance urucum, cochineal carmine, turmeric, saffron, and beet. The dye may be added for instance to the fermentation bath during fermentation such that color is successfully incorporated into the BC layer. Alternatively, the dye may be added to a previously formed BC layer.

The material of the invention also comprises at least one layer of biobased and biodegradable plastic (sometimes referred to herein simply as bioplastic). The bioplastic confers waterproofing (hydrophobic) features to the sheet of BC and allows obtaining a glossy appearance.

Bioplastics contemplated for the present invention are polyesters as well as copolymers thereof. Examples of polyesters include, but are not limited to, polyhydroxyalkanoates (PHAs) and poly(lactic acid) (PLAs). Preferably, the layer (or layers) of bioplastic is transparent, thin and may be cut with the same size than the BC layer.

In a particular embodiment, the average thickness of the at least one bioplastic layer ranges from about 10 µm to about 60 µm such as about 15 µm, 20 µm, 25 µm, 30 µm, 35 µm, 40 µm, 45 µm, 50 µm or 55 µm. In a more particular embodiment, the bioplastic layer is about 15 µm to about 25 µm, and even more particularly 20 µm.

PHA (polyhydroxyalkanoate) is the general term for a range of diverse biodegradable polymers that consist of polyesters of (R)-3-hydroxyalkanoic acids. These polymers are of interest due to a broad range of applications and the fact that they are completely biodegradable. These polymers can be accumulated by some bacteria intracellularly as carbon storage materials. It has been shown that PHA accumulation occurs in bacteria in response to a range of environmental stress factors such as inorganic nutrient limitation. The substrates that are supplied to bacteria to accumulate PHA are divided into two groups 1) PHA related substrates, i.e. alkanoic acids (fatty acids) that resemble the monomers that make up PHA ((R)-3-hydroxyalkanoic acids) and 2) PHA unrelated substrates, which are substrates that do not resemble the monomers that make up PHA e.g. glucose.

In a particular embodiment of the invention, the method for producing PHA comprises (i) culturing in a culture medium one or more bacterial strains which are capable of accumulating PHA and which are preferably selected from a strain of *Alcaligens* (e.g. a strain of *Alcaligens eutrophus* or *Alcaligens latus) or Pseudomonas* (e.g. a *strain of Pseudomonas oleovorans* or *Pseudomonas putida*) and (ii) recovering the PHA produced from the culture medium.

In a particular embodiment, PHA is obtained as disclosed in WO 2009124918. More particularly, the method for producing PHA comprises (i) culturing in a culture medium comprising terephthalic acid and/or a salt thereof and/or an ester thereof one or more bacterial strains which are capable of accumulating PHA from terephthalic acid or a salt or ester thereof and which are selected from *Pseudomonas putida* strain GO16 having the accession number NCIMB 41538, *Pseudomonas putida* strain GO19 having the accession number NCIMB 41537, and *Pseudomonas frederiksbergensis* strain GO23 having the accession number NCIMB 41539; and (ii) recovering the PHA produced from the culture medium.

PHAs are generally classified as short chain length PHAs (scIPHAs), medium chain length PHAs (mcIPHAs) or long chain length PHAs (IcIPHAs), depending upon the number of carbon atoms of the constituting monomers thereof. ScIPHA comprises monomers of C3 - C5, mclPHA comprises monomers of C6 - C14, and IcIPHA comprises monomers of more than 14 carbons (> C14). For the purposes of the present invention, the PHA recovered from the culture medium advantageously comprises mcIPHA, i.e. is made up of repeating units derived from 3-hydroxyalkanoic acid monomers containing 6 carbon atoms (C6) to 14 carbon atoms (C14). More particularly the PHA comprises repeating units of C6, C8, C10, C12 and C14 monomers or repeating units of C8, C10 and C12. Even more particularly, the PHA comprises at least 80%, preferably at least 85%, more preferably at least 90%, most preferably at least 95% medium chain length (mcl) PHA. Preferably, at least 80%, more preferably 85%, even more preferably at least 90%, most preferably at least 95% of the mclPHA comprises repeating units of C8, C10 and C12 monomers. More preferably, the mclPHA comprises repeating units in a respective amount by weight of mclPHA of 15% - 25% C8, 40% - 50% C10 and 30% - 40% C12, preferably as determined using an Agilent 6890N series gas chromatograph (GC) fitted with a 30m × 0.25mm × 0.25µm BP-21 column (J & W Scientific), and further preferably as confirmed using an Agilent 6890N GC fitted with a 5973 series inert mass spectrophotometer, using a 12m × 0.2mm × 0.33µm HP-1 column (Hewlett-Packard). In a preferred embodiment, the amount of C8, C10 and C12 totals 100%. McIPHAs, and particularly mclPHAs comprising monomers selected from C8, C10 and C12 monomers are elastomers and have more desirable properties.

PLA (polyactic acid) is typically made from the sugars in corn starch, cassava or sugarcane. It is biodegradable, carbon-neutral and edible. To transform corn into plastic, corn kernels are immersed in sulfur dioxide and hot water, where its components break down into starch, protein, and fiber. The kernels are then ground and the corn oil is separated from the starch. The starch is comprised of long chains of carbon molecules, similar to the carbon chains in plastic from fossil fuels. Some citric acids are mixed in to form a long-chain polymer (a large molecule consisting of repeating smaller units) that is the building block for plastic. PLA can look and behave like polyethylene (used in plastic films, packing and bottles), polystyrene (Styrofoam and plastic cutlery) or polypropylene (packaging, auto parts, textiles). Minnesota-based NatureWorks is one of the largest companies producing PLA under the brand name Ingeo. In a preferred embodiment, the PLA is about 15 µm to about 25 µm. More particularly, the PLA is about 20 µm thick and may have a density of about 1.24 g/cm³, biobased, biodegradable and compostable, with high transparency and gloss, high mechanical resistance. In a preferred embodiment, the PLA is certified as a 4-star BIOBASED product from Vincotte and 100% compostable according EN13432 from DinCertco.

The layers of BC and bioplastic are bonded/attached by any means that allows keeping both layers together and resists their separation. In an embodiment, the layers of BC and bioplastic are bonded by means of an adhesive layer. This may be carried out by applying an adhesive over the layer of BC (or dyed BC, if a dye has been used for coloring), over the plastic layer or over both. The adhesive is preferably applied over the whole surface of the BC layer (or dyed BC), over the whole surface of the plastic layer or over the whole surfaces of the both layers to be bonded. The adhesive may be applied for instance by spray gun, brush or paint roller. The resulting layered material may be subjected to pressure for a time sufficient (e.g. at least 2, 4, 6 or 8 h) to improve the bonding quality. Pressure can be applied by mechanical means, such as by a press.

Materials useful for the adhesive layer in the present invention may be adhesives commonly used in the leather industry and are preferably selected from water-based adhesives and biopolymer-based adhesives. Water-based adhesives include the aqueous dispersions of homopolymers and copolymers of various polymeric compositions such as polyurethanes, polychloroprenes, latex, etc. In a particular embodiment, the adhesive is a polyurethane or a polychloroprene adhesive.

Alternatively, the layers of BC and bioplastic may be bonded by chemically or mechanically modifying any or both of said layers. For example, the layers of BC and bioplastic may be bonded modifying any or both of said layers using physical adsorption (coating of biomaterial surfaces with cell adhesive proteins like fibronectin, vitronectin, collagen or laminirn, or extracellular matrix resembling molecules such as chitosan and gelatin), chemical treatment (e.g. a commonly used chemical modification method is the alkali hydrolysis of aliphatic polyester surfaces), modification via plasma treatment with a low-pressure gas or photochemical techniques that use high-energy protons to initiate chemical reactions.

The quality assessment on the production of the leather substitute of the invention may be done through physical validations in accordance with accepted standards (EN, ISO, etc.) such as determination of thickness (mm) according to ISO 2589:2003 and determination of tensile strength a (N/mm²) and percentage extension according to ISO 3376:2012.

Further, samples of material may be subjected to the sessile drop wettability test or similar tests to check liquid resistance ability.

The ease of the manufacture method, the non-toxic and biodegradable ingredients used and their availability at industrial scale allow this technology to be implemented in the leather industry on a large scale. The material of the invention finds practical application as leather substitute in a variety of areas such as the textile, clothing, apparel, footwear, furniture and transport sectors. The invention is also directed to footwear, comprising the leather substitute material as herein proposed.

The following examples are merely illustrative of certain embodiments of the invention and cannot be considered as restricting it in any way.

### EXAMPLES

### Example 1 - Preparation of leather substitute with PHA

A layer of bacterial cellulose was obtained from a symbiotic culture of bacteria and yeast (SCOBY) composed of *Komagataeibacter xylinus* bacteria and two yeasts: *Zygosaccharomyces rouxii* and *Candida sp.* A green tea infusion was prepared by placing 8 g of tea leaves in 2 liters of boiling water. After about 15 minutes, tea leaves were withdrawn and sucrose 80 g/l was added as carbon source. Once the temperature was below 30 °C, the mixture was thrown into a 0.09 m² (i.e. 1 square foot) bucket. Then, the SCOBY was added in suspension (10% v/v) and the buckets were covered with a filter paper. Fermentation took place in 15 days at a temperature of 30 +/- 3°C. Then, the sheets of BC were taken and washed with soapy water several times during 12 h. The BC sheets were immersed in a 2% potassium hydroxide solution for about 90 min at 25 °C with oscillating agitation for the elimination of impurities and cell debris, followed by a neutralization treatment with acetic acid (pH 3.0) for 30 minutes at 25 °C. Subsequently, the sheets of BC were dried vertically for 24 hours at 35 °C.

A layer of mclPHA comprising C8, C10 and C12 monomers was bonded to the layer of BC by means of adhesive, by application of pressure for at least 6 hours.

The resulting material was suitable as leather substitute for the footwear industry.

### Example 2 - Preparation of leather substitute with PLA

Bacterial cellulose was obtained as disclosed in example 1 but using a layer of PLA. The layers were bonded as disclosed in example 1.

The resulting material was suitable as leather substitute for the footwear industry.

## Claims

1. A footwear which comprises a material comprising:
- a layer of bacterial cellulose (BC); and
- a layer of a biobased and biodegradable plastic selected from the group consisting of polyesters or copolymers thereof;
wherein both layers are attached each other.

2. The footwear according to claim 1, wherein the BC layer is obtained from a symbiotic culture of bacteria and yeast (SCOBY) comprising at least one type of BC-producing bacteria and at least one yeast type.

3. The footwear according to claim 2, wherein the SCOBY comprises at least one bacterial strain of *Komagataeibacter xylinus* and the yeasts *Zygosaccharomyces rouxii* and *Candida sp.*

4. The footwear according to any one of the preceding claims, wherein the biobased and biodegradable plastic is compostable.

5. The footwear according to any one of the preceding claims, wherein the biobased and biodegradable plastic is selected from the group consisting of polyhydroxyalkanoate (PHA) and poly(lactic acid) (PLA) or a copolymer thereof.

6. The footwear according to claim 5, wherein the polyhydroxyalkanoate (PHA) is a medium chain length polyhydroxyalkanoate (mcIPHA) with repeating units of C8, C10 and C12 monomers.

7. The footwear according to claim 5, wherein the PLA is about 10 µm to about 60 µm thick, and preferably transparent and glossy.

8. The footwear according to any one of the preceding claims wherein the BC layer and the biobased and biodegradable plastic layer are attached each other by means of an intermediate layer such as an adhesive layer based on polychloroprene or polyurethane.

9. A method for preparing the footwear as defined in any one of the preceding claims, said method comprising the preparation of the material by a process which comprises the following steps:
- providing a layer of bacterial cellulose (BC);
- providing a layer of biobased and biodegradable plastic selected from the group consisting of polyesters or copolymers thereof; and
- attaching both layers.

10. The method according to claim 9, wherein the layer of BC is obtained by a process comprising: (i) culturing in a culture medium a symbiotic culture of bacteria and yeast (SCOBY) comprising at least one bacterial strain of *Komagataeibacter xylinus* and the yeasts *Zygosaccharomyces rouxii* and *Candida sp* and (ii) recovering the BC produced from the culture medium.

11. Use as leather substitute of a material comprising:
- a layer of bacterial cellulose (BC); and
- a layer of a biobased and biodegradable plastic selected from the group consisting of polyesters or copolymers thereof;
wherein both layers are attached each other.

12. Use according to claim 11, wherein the BC layer is obtained from a symbiotic culture of bacteria and yeast (SCOBY) comprising at least one type of BC-producing bacteria and at least one yeast type.

13. Use according to any one of claims 11 or 12, wherein the biobased and biodegradable plastic is compostable.

14. Use according to any one of claims 11 to 13, wherein: the biobased and biodegradable plastic is selected from the group consisting of polyhydroxyalkanoate (PHA) and poly(lactic acid) (PLA) or a copolymer thereof.

15. Use according to any one of claims 11 to 14, wherein the BC layer and the biobased and biodegradable plastic layer are attached each other by means of an intermediate layer such as an adhesive layer based on polychloroprene or polyurethane.

## Patentansprüche

1. Schuhwerk, das ein Material umfasst, das Folgendes umfasst:
- eine Schicht aus bakterieller Cellulose (BC); und
- eine Schicht aus einem biobasierten und biologisch abbaubaren Kunststoff, ausgewählt aus der Gruppe, die aus Polyestern oder deren Copolymeren besteht; wobei beide Schichten miteinander verbunden sind.

2. Schuhwerk nach Anspruch 1, wobei die BC-Schicht aus einer symbiotischen Bakterien- und Hefekultur (SCOBY) gewonnen wird, die mindestens eine Art von BCproduzierenden Bakterien und mindestens eine Hefeart umfasst.

3. Schuhwerk nach Anspruch 2, wobei der SCOBY mindestens einen Bakterienstamm von *Komagataeibacter xylinus* und die Hefen *Zygosaccharomyces rouxii* und *Candida sp.* umfasst.

4. Schuhwerk nach einem der vorhergehenden Ansprüche, wobei der biobasierte und biologisch abbaubare Kunststoff kompostierbar ist.

5. Schuhwerk nach einem der vorhergehenden Ansprüche, wobei der biobasierte und biologisch abbaubare Kunststoff ausgewählt ist aus der Gruppe bestehend aus Polyhydroxyalkanoat (PHA) und Poly(milchsäure) (PLA) oder einem Copolymer davon.

6. Schuhwerk nach Anspruch 5, wobei das Polyhydroxyalkanoat (PHA) ein Polyhydroxyalkanoat mittlerer Kettenlänge (mclPHA) mit wiederkehrenden Einheiten aus C8-, C10- und C12-Monomeren ist.

7. Schuhwerk nach Anspruch 5, wobei das PLA etwa 10 µm bis etwa 60 µm dick und vorzugsweise transparent und glänzend ist.

8. Schuhwerk nach einem der vorhergehenden Ansprüche, bei dem die BC-Schicht und die biobasierte und biologisch abbaubare Kunststoffschicht durch eine Zwischenschicht wie eine Klebeschicht auf Basis von Polychloropren oder Polyurethan miteinander verbunden sind.

9. Verfahren zur Herstellung von Schuhwerk, wie in einem der vorhergehenden Ansprüche definiert, wobei das Verfahren die Herstellung des Materials durch ein Verfahren umfasst, das die folgenden Schritte umfasst:
- Bereitstellen einer Schicht aus bakterieller Cellulose (BC);
- Bereitstellen einer Schicht aus biobasiertem und biologisch abbaubarem Kunststoff, ausgewählt aus der Gruppe bestehend aus Polyestern oder deren Copolymeren; und
- Verbinden beider Schichten.

10. Verfahren nach Anspruch 9, wobei die BC-Schicht durch ein Verfahren erhalten wird, das Folgendes umfasst: (i) Züchten einer symbiotischen Bakterien- und Hefekultur (SCOBY), die mindestens einen Bakterienstamm von *Komagataeibacter xylinus* und die Hefen *Zygosaccharomyces rouxii* und *Candida sp* umfasst, in einem Kulturmedium und (ii) Gewinnen der erzeugten BC aus dem Kulturmedium.

11. Verwendung eines Materials als Lederersatz, umfassend:
- eine Schicht aus bakterieller Cellulose (BC); und
- eine Schicht aus einem biobasierten und biologisch abbaubaren Kunststoff, ausgewählt aus der Gruppe bestehend aus Polyestern oder deren Copolymeren; wobei beide Schichten miteinander verbunden sind.

12. Verwendung nach Anspruch 11, wobei die BC-Schicht aus einer symbiotischen Bakterien- und Hefekultur (SCOBY) erhalten wird, die mindestens eine Art von BCproduzierenden Bakterien und mindestens eine Hefeart umfasst.

13. Verwendung nach einem der Ansprüche 11 oder 12, wobei der biobasierte und biologisch abbaubare Kunststoff kompostierbar ist.

14. Verwendung nach einem der Ansprüche 11 bis 13, wobei: der biobasierte und biologisch abbaubare Kunststoff ausgewählt ist aus der Gruppe bestehend aus Polyhydroxyalkanoat (PHA) und Poly(milchsäure) (PLA) oder einem Copolymer davon.

15. Verwendung nach einem der Ansprüche 11 bis 14, wobei die BC-Schicht und die Schicht aus biobasiertem und biologisch abbaubarem Kunststoff mittels einer Zwischenschicht, wie einer Klebeschicht auf Basis von Polychloropren oder Polyurethan, miteinander verbunden sind.

## Revendications

1. Une chaussure qui comprend un matériau comprenant :
- une couche de cellulose bactérienne (BC) ; et
- une couche d'un plastique biosourcé et biodégradable choisi dans le groupe constitué des polyesters ou des copolymères de ceux-ci ;
les deux couches étant fixées l'une à l'autre.

2. La chaussure selon la revendication 1, dans laquelle la couche de BC est obtenue à partir d'une culture symbiotique de bactéries et de levures (SCOBY) comprenant au moins un type de bactérie productrice de BC et au moins un type de levure.

3. La chaussure selon la revendication 2, dans laquelle le SCOBY comprend au moins une souche bactérienne de *Komagataeibacter xylinus* et les levures *Zygosaccharomyces rouxii* et *Candida sp.*

4. La chaussure selon l'une quelconque des revendications précédentes, dans laquelle le plastique biosourcé et biodégradable est compostable.

5. La chaussure selon l'une quelconque des revendications précédentes, dans laquelle le plastique biosourcé et biodégradable est choisi dans le groupe constitué du polyhydroxyalcanoate (PHA) et du poly(acide lactique) (PLA) ou d'un copolymère de ceux-ci.

6. La chaussure selon la revendication 5, dans laquelle le polyhydroxyalcanoate (PHA) est un polyhydroxyalcanoate de longueur de chaîne moyenne (mcIPHA) avec des unités répétitives de monomères C8, C10 et C12.

7. La chaussure selon la revendication 5, dans laquelle le PLA a une épaisseur d'environ 10 µm à environ 60 µm, et de préférence transparent et brillant.

8. La chaussure selon l'une quelconque des revendications précédentes dans laquelle la couche de BC et la couche plastique biosourcée et biodégradable sont fixées l'une à l'autre au moyen d'une couche intermédiaire telle qu'une couche adhésive à base de polychloroprène ou de polyuréthane.

9. Un procédé de préparation de chaussures telles que définies dans l'une quelconque des revendications précédentes, ledit procédé comprenant la préparation du matériau par un procédé qui comprend les étapes suivantes :
- fournir une couche de cellulose bactérienne (BC) ;
- fournir une couche de plastique biosourcé et biodégradable choisi dans le groupe constitué de polyesters ou de copolymères de ceux-ci ; et
- fixer les deux couches.

10. Le procédé selon la revendication 9, dans lequel la couche de BC est obtenue par un procédé comprenant : (i) la culture dans un milieu de culture d'une culture symbiotique de bactéries et de levures (SCOBY) comprenant au moins une souche bactérienne de *Komagataeibacter xylinus* et les levures *Zygosaccharomyces rouxii* et *Candida sp* et (ii) récupérer la BC produite à partir du milieu de culture.

11. Utilisation comme substitut du cuir d'un matériau comprenant :
- une couche de cellulose bactérienne (BC) ; et
- une couche d'un plastique biosourcé et biodégradable choisi dans le groupe constitué des polyesters ou des copolymères de ceux-ci ;
les deux couches étant fixées l'une à l'autre.

12. Utilisation selon la revendication 11, dans laquelle la couche de BC est obtenue à partir d'une culture symbiotique de bactéries et de levures (SCOBY) comprenant au moins un type de bactérie productrice de BC et au moins un type de levure.

13. Utilisation selon l'une quelconque des revendications 11 ou 12, dans laquelle le plastique biosourcé et biodégradable est compostable.

14. Utilisation selon l'une quelconque des revendications 11 à 13, dans laquelle : le plastique biosourcé et biodégradable est choisi dans le groupe constitué du polyhydroxyalcanoate (PHA) et du poly(acide lactique) (PLA) ou d'un copolymère de ceux-ci.

15. Utilisation selon l'une quelconque des revendications 11 à 14, dans laquelle la couche de BC et la couche plastique biosourcée et biodégradable sont fixées l'une à l'autre au moyen d'une couche intermédiaire telle qu'une couche adhésive à base de polychloroprène ou de polyuréthane.
